# EUROPÄISCHE PATENTSCHRIFT

(11) **EP 3 069 124 B1**
(45) Veröffentlichungstag und Bekanntmachung des Hinweises auf die Patenterteilung: **18.08.2021**
(21) Anmeldenummer: 14796754.1
(22) Anmeldetag: 11.11.2014
(51) Int. Cl.: G01N 21/53, B01D 1/22, B01D 3/08, C12M 1/21, G01N 21/85, G01N 29/032, G01N 29/34

(54) **VERDAMPFUNGSSYSTEM UMFASSEND EINE VORRICHTUNG UND VERFAHREN ZUR ERKENNUNG EINER SCHAUMENTWICKLUNG**
EVAPORATION SYSTEM COMPRISING A DEVICE AND METHOD FOR RECOGNIZING THE DEVELOPMENT OF FOAM
SYSTÈME D'ÉVAPORATION COMPRENANT UN DISPOSITIF ET PROCÉDÉ DESTINÉS À LA DÉTECTION D'UN DÉVELOPPEMENT DE MOUSSE

(30) Priorität: 13.11.2013 EP 13192614
(43) Veröffentlichungstag der Anmeldung: 21.09.2016
(73) Patentinhaber: Büchi Labortechnik AG, 9230 Flawil (CH)
(72) Erfinder: BÜRKI, Martin, CH-9248 Bichwil (CH)
(74) Vertreter: Hepp Wenger Ryffel AG
(86) Internationale Anmeldenummer: PCT/EP2014/074225
(87) Internationale Veröffentlichungsnummer: WO 2015/071237

(56) Entgegenhaltungen:
- EP-A2- 2 570 784
- DE-A1- 10 054 924
- DE-A1- 10 114 434
- DE-A1- 19 617 106
- JP-U- H 057 301
- US-A- 5 453 832
- US-A1- 2011 273 710
- US-B1- 6 435 710

## Beschreibung

Die Erfindung betrifft ein Verdampfungssystem umfassend ein Prozessgefäss und eine Vorrichtung zur Erkennung einer Schaumentwicklung, und ein Verfahren zur Erkennung einer Schaumentwicklung gemäss den Oberbegriffen der unabhängigen Ansprüche.

Beim Betrieb gängiger Verdampfungssysteme wird ein zu verdampfendes Medium durch Erhitzen eines Prozessgefässes aus der Fest- und/oder Flüssigphase in die Dampfphase überführt. In vielen Fällen wirft das zu verdampfende Medium Blasen auf bzw. ein Schaum entwickelt sich auf der Oberfläche des Mediums. Dies ist zumeist abhängig vom zu verdampfenden Medium und von angelegten Verfahrensparametern. Vorrichtungen zur Erkennung einer Schaumentwicklung werden beim Eindampfen, Verdampfen und Destillieren von Flüssigkeiten, Flüssigkeitsgemischen, Stoffen und Stoffgemischen eingesetzt. Dabei ist es erforderlich eine aufkommende Schaumentwicklung zu erkennen und gegebenenfalls zu verhindern oder ein Überschäumen eines Schaums zu verhindern. Es soll vermieden werden, dass Schaum in eine Dampfdurchführung oder in einen Auffangbehälter eines Destillats eintritt. Insbesondere beim Betrieb eines Rotationsverdampfers, ist eine Schaumentwicklung zu vermeiden, da durch das Überschäumen und das Eintreten des Schaums in den Auffangbehälter das Destillat verunreinigt wird, bzw. Stoffe des Rückstandes verloren gehen.

Vorrichtungen und Verfahren zur reinen Vermeidung und zur Erkennung mit darauf folgenden Massnahmen zur Reduktion einer Schaumentwicklung sind bekannt. Verfahren und Vorrichtungen zur Vermeidung einer Schaumentwicklung nutzen beispielsweise vordefinierte Zugabeintervalle eines zu verdampfenden Mediums in einen Verdampferkolben, womit eine Schaumentwicklung vermieden wird. Dies ist in US 2003/0111185 A1 gezeigt. Optische Schaumsensoren zur Erkennung einer Schaumentwicklung sind in DE 10 2010 007 559 A1 beschrieben. Dabei ist ein optisches System bestehend aus einer Lichtquelle und einem Sensor in einem Fenster angeordnet, welches in einer Wand eines Bioreaktorbehälters eingelassen ist. Bei Erkennung eines aufkommenden Schaums mittels eines reflektierten Kontrollsignals werden Antischaumagenzien, welche der Schaumentwicklung entgegen wirken, zugegeben.

Vorgenannte Vorrichtung, welche auf einer optischen Erkennung einer Schaumentwicklung basiert, verwendet jedoch spezielle Behälter, insbesondere mit einer speziellen Behälterwand. Dabei müssen ein oder mehrere Fenster und/oder Öffnungen in der Wand des Behälters eingelassen sein, in welche Elemente eines optischen Systems eingelassen sind, um eine optische Erkennung durchführbar zu machen. Während des Betriebs kann es zu Ablagerungen und Verschmutzungen der Fenster und/oder Öffnungen kommen, was die Erkennung einer Schaumentwicklung nachteilig beeinträchtigt. Zudem ist eine Verwendung des Systems mit rotierenden Behältern ausgeschlossen.

US 5,453,832 A beschreibt eine Vorrichtung zur Bestimmung der Trübheit von flüssigen Proben, welche feste Partikel und Gasblasen enthalten. Die Vorrichtung ist im Strömungsweg einer Flüssigkeit, deren Trübheit ermittelt werden soll, angeordnet.

US 2011/273710 A1 beschreibt eine Kopfeinheit für einen Trübheitssensor sowie einen Trübheitssensor.

EP 2 570 784 A2 beschreibt einen Füllhöhendetektor eines Gefässes sowie ein Verfahren zur Schaumdetektion in einem Gefäss. Der Füllhöhendetektor ist durch eine Wandung des Gefässes in das Innere des Gefässes eingebracht.

US 6,435,710 B1 beschreibt eine Vorrichtung zur Schaumerkennung, welche auf der Ermittlung einer Temperaturdifferenz einer Messoberfläche bei Inkontakttreten mit einem Schaum basiert.

DE 100 54 924 A1 zeigt ein Verfahren zur Erfassung der Zellöffnung während der Schaumbildung in schaumbildenden Substanzen.

DE 101 14 434 A1 beschreibt eine Vorrichtung mit zwei extern angeordneten Schaumsensoren zur Erfassung einer Schaumbildung in einem Rotationsgefäss.

Die vorgenannten Vorrichtungen sind nachteilig, da sie die Trübheit einer Flüssigkeit messen, wobei der Trübheitsfühler in der Flüssigkeit positioniert ist; oder, da sie in die Gefässwand eingelassen sind oder ausserhalb des Gefässes positioniert sind, oder auf einem Wärmeaustausch zur Bestimmung basieren.

Es ist daher die Aufgabe der Erfindung die Nachteile des Standes der Technik zu überwinden. Insbesondere ist es Aufgabe der Erfindung, ein Verdampfungssystem umfassend ein Prozessgefäss und eine Vorrichtung zur Erkennung einer Schaumentwicklung, und ein Verfahren zur Erkennung einer Schaumentwicklung in einem Prozessgefäss eines Verdampfungssystems bereitzustellen, welche geringe Herstellungskosten aufweisen, welche ein kostengünstiges Nachrüsten von Verdampfungssystemen ermöglichen, welche eine verbesserte Leistungsfähigkeit aufweisen und für den Anwender einfach in der Anwendung sind. Diese Aufgaben werden durch die in den unabhängigen Patentansprüchen definierten Merkmale gelöst.

Die Erfindung betrifft ein Verdampfungssystem umfassend ein Prozessgefäss und eine Vorrichtung zur Erkennung einer Schaumentwicklung umfassend einen Detektor zum Erfassen eines elektromagnetischen und/oder eines akustischen Signals. Die Erfindung zeichnet sich dadurch aus, dass die Vorrichtung im Prozessgefäss anordenbar oder angeordnet ist. Auf diese Weise wird ein kostengünstiges Nachrüsten von Systemen gewährleistet, da die Vorrichtung zur Erkennung einer Schaumentwicklung im Prozessgefäss angeordnet und innerhalb dieses betrieben werden kann. Somit ist kein speziell an die Schaumerkennung angepasstes Prozessgefäss, z.B. mit einem Fenster und/oder einer Öffnung in der Gefässwand, erforderlich. Zudem kann eine Schaumentwicklung mittels des Detektors innerhalb des Prozessgefässes also nahe beim Entstehen erkannt werden. Eine Verschmutzung der Gefässwand hat somit keinen negativen Einfluss.

Die Vorrichtung umfasst zusätzlich eine Auswerteinheit zum Auswerten des vom Detektor erfassten Signals. Die Vorrichtung stellt somit schon das ausgewertete Signal zur Steuerung bzw. zur Weiterverarbeitung zur Verfügung. Anwendungsübliche Auswerteinheiten umfassen einen Verstärker sowie einen Korrelator. Mittels des Verstärkers wird das Signal auf einen Amplitudenwert angehoben, so dass dessen weitere Bearbeitung einfach realisierbar ist. Der Korrelator filtert einen Störanteil des Signals weg. Alternativ kann ein Hochpassfilter, ein Tiefpassfilter oder ein Bandpassfilter, je nach verwendetem Signaltyp, verwendet werden. In der einfachsten Ausführung wird dieses Signal mittels eines Komparators digitalisiert, so dass lediglich die An-/Abwesenheit von Schaum dargestellt wird. Selbstverständlich sind auch komplexere Auswerteinheiten denkbar. Vorzugsweise ist die Auswerteinheit auf einer einzigen Leiterplatte, insbesondere auf derselben Leiterplatte wie der Detektor angeordnet.

Die Auswerteinheit verarbeitet ein erfasstes Signal vorort in der Vorrichtung und dementsprechend wird ein ausgewertetes Signal weitergeleitet. Das ausgewertete Signal kann analog oder digitalisiert weitergeleitet werde. Die erfassten Signale sind per se empfindlich bzw. störanfällig. Dieser Nachteil wird durch die integrierte Auswerteinheit vermieden, da die Auswertung vor einem Weiterleiten eines Signals stattfindet. Durch die Anordnung in einer abgeschlossenen Einheit kann des Weiteren eine einfache Schnittstelle zur Weiterleitung des bereits ausgewerteten und gegebenenfalls digitalisierten Signals zur Verfügung gestellt werden. Damit ist die Handhabung der Vorrichtung ähnlich oder gleich wie bei anderen bekannten Sensoren, beispielsweise Temperatur- oder Drucksensoren.

Zur Datenübertragung des ausgewerteten Signals kann eine diskrete Signalleitung oder aber auch eine andere Form der Datenübertragung vorgesehen sein. Entsprechend kann die Vorrichtung eine Datenschnittstelle zur Übertragung des ausgewerteten Signals umfassen.

Die Vorrichtung kann zusätzlich eine Quelle zum Erzeugen des elektromagnetischen und/oder des akustischen Signals aufweisen. Damit kann ein vordefiniertes elektromagnetisches und/oder akustisches Signal generiert werden, welches mittelbar und/oder unmittelbar vom Detektor innerhalb des Prozessgefässes detektiert wird. Die Vorrichtung ist somit unabhängig von externen oder zusätzlichen Quellen. Die Zuverlässigkeit der Schaumerkennung wird somit verbessert.

Das von der Quelle erzeugte, insbesondere gepulste Signal kann für das menschliche Auge und/oder das menschliche Gehör nicht wahrnehmbar sein. Das Signal kann auf der Verwendung von Infrarotlicht basieren. Dabei weist die Quelle ein Sendefenster beispielsweise im Bereich von 850 nm auf. Der Detektor weist einen Empfangsbereich auf, welcher das Sendefenster umfasst, insbesondere zwischen 730 bis 1010 nm. Selbstverständlich kann das Signal als elektromagnetische Welle, insbesondere im Radiofrequenzbereich (Funkwellen) verschiedener Frequenzbänder, oder im Hochfrequenzbereich, insbesondere im Bereich 0,1 bis 100 GHz, ausgesendet werden. Selbstverständlich kann das Signal auch als Ultraschall ausgesendet werden. Durch die Verwendung eines gepulsten Signals kann das Signal-Rausch-Verhältnis (signal-to-noise-ratio) verbessert werden.

Durch die Verwendung eines schmalbandigen Signals mit einem entsprechenden Filter vor dem Detektor kann das Signal-Rausch Verhältnis (signal-to-noise) verbessert werden.

Ausserdem kann das Signal auf der Seite der Quelle moduliert und auf der Seite des Detektors entsprechend demoduliert werden, was ebenfalls zu einem verbesserten Signal-Rausch Verhältnis (signal-to-noise-ratio) führt.

Schliesslich kann ein quasizufälliges Signal (spread spectrum) verwendet werden. In Kombination mit einem Korrelator nach dem Detektor wird wiederum das Signal-Rausch Verhältnis verbessert und die Störfestigkeit gegenüber Fremdsignalen verbessert.

Anwendungsspezifische Filter oder Vorfilter, die dem Detektor vorgelagert oder nachgeschaltet sind, können auf das charakteristische zufällige Reflexionsverhalten eines Schaums optimiert sein. Dabei ist die einfachste Ausführung die Verwendung eines Hochpassfilters.

Bei der Quelle kann es sich um eine Leuchtdiode zum Aussenden von schmalbandigem Licht (LED, light-emitting-diode), einen Infrarot-Transmitter, eine Laserdiode, einen Piezolautsprecher oder einen piezoelektrischen Quarz- oder Keramikschwinger handeln. Entsprechend wäre der Detektor beispielsweise als Photodiode, Phototransistor, Photowiderstand (LDR, light dependent resistor), Infrarotempfänger oder Piezosensor (Richtmikrophon) ausgestaltet. Der Detektor kann als Kombination mit einem kapazitivem Detektor ausgestaltet sein, beispielsweise als Kombination aus Infrarot-Detektor und kapazitivem Detektor.

Selbstverständlich können auch eindimensionale und/oder zweidimensionale (1D und 2D) Sensoren, beispielsweise CCD (chargecoupled device) Bildsensoren zur Erkennung von Bewegung im Prozessgefäss umfasst sein. Dabei weisen vorzugsweise die Bildsensoren Bereiche mit ausreichender Tiefenschärfe auf, um das Erkennen einer Bewegung, insbesondere einer Schaumentwicklung, zu erfassen.

Die Vorrichtung kann eine Einheit aufweisen, in welcher die Quelle und der Detektor angeordnet sind. Dabei kann die Einheit als eine einzelne Baugruppe ausgestaltet sein, wobei Kombinationssensoren verwendet werden können. Innerhalb der Einheit können die Quelle und der Detektor nebeneinander oder voneinander beabstandet angeordnet sein. Somit können, im Falle dass die Quelle und der Detektor nebeneinander positioniert sind, vom Schaum reflektierte Signale detektiert werden. Sind die Quelle und der Detektor beabstandet voneinander angeordnet, vorzugsweise beabstandet in einem Bereich von 1 bis 5 cm werden überwiegend durch den Schaum transmittierte Signale detektiert. Letzteres setzt voraus, dass ein Bereich der Vorrichtung, welcher entweder die Quelle oder den Detektor umfasst, in einer Zone des Prozessgefässes, in welcher sich Schaum befindet oder in welcher sich Schaum entwickeln wird, positioniert ist.

Die Einheit der Vorrichtung kann stabförmig ausgestaltet sein, insbesondere gekapselt in einem für das Signal transparenten Stab, insbesondere aus Glas oder Kunststoff, angeordnet sein. Auf diese Weise kann die Einheit optimal innerhalb eines Prozessgefässes, insbesondere innerhalb eines länglich ausgestalteten Prozessgefässes, positioniert werden und/oder durch weiterführende Bauteile, insbesondere bei einem Verdampfungssystem, wie beispielsweise eine Dampfdurchführung, hindurchreichen. Die im Stab gekapselte Einheit ist vorteilig dahingehend, dass sie ein einfaches Reinigen der Einheit ermöglicht. Beispielsweise ist die Einheit in ein für das Signal transparentes Rohr, bevorzugt ein Glasrohr oder einem Kunststoffrohr, eingeschoben. Zudem reinigt rückfliessendes Kondensat die gekapselte Einheit. Vorteilhaft ist dabei ebenso, dass ein Kontakt des Mediums im Prozessgefäss aufgrund der Kapselung nur mit dem Glas oder dem Kunststoffs des Rohrs stattfinden kann.

Die Quelle und der Detektor können derart relativ zueinander angeordnet sein, dass der Detektor ein von einem Schaum reflektiertes Signal und/oder ein durch den Schaum transmittiertes Signal detektiert. Auf diese Weise können Signale, die nach Interaktion mit dem Schaum reflektiert oder transmittiert werden, vom Detektor erfasst werden. Anhand des empfangenen Signals wird so eine Schaumentwicklung erkannt. Vorgenannter Schaum kann von einem Medium aus einem Stoff oder einem Stoffgemisch und/oder einer Flüssigkeit oder einem Flüssigkeitsgemisch, insbesondere von einem Lösungsmittel oder einem Lösungsmittelgemisch umfassend gängige Lösungsmittel, herrühren.

Beispielhafte Lösungsmittel, welche üblicherweise in Verdampfungssystemen benutzt werden, sind Acteon, Benzol, Chlorbenzol, 1,2-Dichlorethan, Dichlormethan, Diethylether, Dioxan, Essigsäure, Ethanol, Ehylacetat, Heptan, Hexan, Methanol, Pentan, n-Propylalkohol, Tetrachlorethylen, Toluol, Trichlorethylen, Trichlormethan, Wasser und Xylole. Weitere gängige Lösungsmittel sind dem Fachmann bekannt.

Vor dem Detektor kann eine Blende, ein Tubus, eine Optik und/oder einen Filter zum Abschirmen und Filtern unerwünschter Streusignale angeordent sein. Ebenso ist die Verwendung von Gittern und/oder Masken zusätzlich oder alternativ möglich. Eine solche Anordnung ist auch vor der Quelle denkbar. Auf diese Weise können das empfangene Signal und/oder das ausgesendete Signal anforderungsgemäss gefiltert werden. Insbesondere eine Detektion von Streustrahlung und unerwünschte Reflexionen werden verringert. Dies resultiert in einem verbesserten Signal-Rausch-Verhältnis. Ausserdem kann bei reflektiven Systemen ein direktes, transmittives Übersprechen von der Quelle zum Detektor verhindert werden.

Bevorzugt kann die Quelle als eine Infrarot-LED (light-emitting-diode) einen Abstrahlwinkel von 30° (±15°) aufweisen. Entsprechend handelt es sich bei dem Detektor um einen Phototransistor mit Blende und Ifrarot-Filter.

Die Vorrichtung kann zusätzlich einen Temperatursensor zum Erfassen einer Temperatur im Prozessgefäss und/oder in einem Verdampfungssystem umfassen. Die erfasste Temperatur kann zur Steuerung oder zur Reglung des Betriebs des Verdampfungssystems berücksichtigt werden, indem prozessspezifische Parameter auf vordefinierte Werte eingestellt werden. Prozessspezifische Parameter eines Verdampfungssystems umfassen eine Temperatur einer Heizvorrichtung, eine Rotationsgeschwindigkeit des Prozessgefässes und/oder einen angelegten Druck im System. Auf diese Weise kann das Verdampfungssystem auf optimale Betriebsbedingungen zum Verdampfen eines Mediums eingestellt werden. Ein zusätzlicher separater Temperatursensor wird nicht benötigt.

Des Weiteren können die Quelle und der Detektor als ein Distanzmesssystem ausgestaltet sein. Derartige Systeme sind beispielsweise als Time-of-Flight Laserdistanzsensoren bekannt. In diesem Fall kann die Distanz zu einem ersten Hindernis, welches die Gefässwand ist, wenn kein Schaum vorhanden ist, gemessen werden. Bei auftretendem Schaum wird diese gemessene Distanz verkürzt und entsprechend ein Schaum detektiert. Zur Verarbeitung der Distanzmessungen können Distanzlimitierungen herangezogen werden, bspw. liegen diese unter der Distanz zur Gefässwand. Eine Verarbeitung wie bei konventionellen Systemen mit Bandpass und Bewertungsfunktion sind ebenfalls möglich. Die Messsignale werden im Anschluss auf Plausibilität überprüft, um Reflexionen von Verunreinigungen, die zu fehlerhaften Ergebnissen führen können, auszuschliessen. Dies ist insbesondere mittels Komparatoren erreichbar; dies jeweils für eine Minimaldistanz und für eine Maximaldistanz. Eine Schaumerkennung erfolgt demnach insbesondere nur dann, wenn das Distanzsignal zwischen der Minimal- und der Maximaldistanz liegt. Dem Komparator kann zur Vermeidung von Störungen ein Tiefpassfilter vorgelagert sein.

Derartige Distanzmesssysteme sind bei grossen Sensoren (10 x 5 x 5 cm³) bekannt, welche beispielsweise zur Füllstandsanzeige bei Industrieanwendungen wie in Öltanks verwendet werden. Diese sind jedoch üblicherweise hinter einer Glaswand positioniert und waren bisher nicht auf Laborgeräte, insbesondere Verdampfungssysteme, anwendbar.

Durch die Verwendung der Vorrichtung innerhalb des Prozessgefässes kann ein Schaum und/oder eine Schaumentwicklung direkt im Prozessgefäss erkannt und frühzeitig reagiert werden. Störeinflüsse werden somit reduziert.

Das Verdampfungssystem kann ein Rotationsverdampfer sein.

Die Vorrichtung kann statisch oder mit dem Verdampferkolben rotierend angebracht sein. Bei einer bevorzugten Anbringung ist die Vorrichtung an einem statischen Element des Verdampfungssystems, insbesondere des Rotationsverdampfers, angebracht und kann durch eine Dampfdurchführung bis in ein Prozessgefäss hinein reichen. Auf diese Weise können Verdampfungssysteme, insbesondere Rotationsverdampfer, mit einer Vorrichtung nachgerüstet werden.

Das Verdampfungssystem kann Mittel aufweisen, um Schaum im Prozessgefäss zu reduzieren. Vorgenannte Mittel umfassen eine Vorrichtung zur Erhöhung des Drucks im Prozessgefäss, zum Senken einer Prozesstemperatur, insbesondere der Temperatur einer Heizvorrichtung, zum Verändern der Rotationsgeschwindigkeit eines Prozessgefässes und/oder zum Eingeben eines Antischaummittels. Durch die vorgenannten Mittel bzw. Massnahmen kann ein erkannter Schaum reduziert werden.

Ein weiterer Aspekt der Erfindung betrifft ein Verfahren zum Erkennen einer Schaumentwicklung in einem Prozessgefäss eines Verdampfungssystems gemäss einem der Ansprüche bis 9.

Dabei umfasst das Verfahren die Schritte
i) Erfassen eines elektromagnetischen und/oder akustischen Signals mittels des im Prozessgefäss angeordneten Detektors der Vorrichtung, und
ii) Auswerten des erfassten Signals mit der Auswerteinheit der Vorrichtung nach vorgegebenen Kriterien zur Erkennung einer Schaumentwicklung.

Durch das Anordnen der Vorrichtung im Prozessgefäss kann ein Schaum und/oder eine Schaumentwicklung direkt im Prozessgefäss erkannt und frühzeitig reagiert werden. Störeinflüsse werden reduziert. Die Kriterien umfassen beispielsweise Veränderungen des Signals und/oder der Signalstärken, insbesondere des Sendeimpulses und/oder Ausblenden von regelmässigen Störungen.

Das Verfahren kann den Schritt des Aussendens eines elektromagnetischen und/oder akustischen Signals von einer Quelle, welche im Prozessgefäss angeordnet ist, umfassen. Auf diese Weise kann ein ausgesendetes Signal, welches von Schaum und/oder einem aufkommenden Schaum verändert wird, durch den Detektor der Vorrichtung erfasst werden. Die Detektion von Schaum ist dadurch unabhängig von einer externen Quelle und entsprechend sind weniger Störeinflüsse vorhanden.

Die Quelle kann dabei ein, insbesondere gepulstes, Signal im Infrarotbereich, insbesondere in einem Wellenlängenbereich von 700 bis 1400 nm, insbesondere im Bereich von 850 nm, und/oder Schallwellen, insbesondere Ultraschallwellen im Frequenzbereich von 20 kHz bis 1 GHz ausstrahlen. Der Detektor weist einen Empfangsbereich auf, welcher den Bereich des ausgesendeten Signals umfasst, und liegt beispielsweise im Bereich von 730 bis 1010 nm. Durch die Verwendung von gepulsten Signalen wird das Signal-Rausch-Verhältnis (signal-to-noise-ratio) verbessert. Selbstverständlich kann das Signal als elektromagnetische Welle, insbesondere im Radiofrequenzbereich (Funkwellen) verschiedener Frequenzbänder, oder im Hochfrequenzbereich, insbesondere im Bereich 0,1 bis 100 GHz, ausgesendet werden. Der Detektor ist entsprechend anzupassen. Bei der Quelle kann es sich um eine Leuchtdiode zum Aussenden von schmalbandigem Licht (LED, light-emitting-diode), einen Infrarot-Transmitter, eine Laserdiode, einen Piezolautsprecher oder einem piezoelektrischen Quarz- oder Keramikschwinger, und bei dem Detektor um eine Photodiode, einen Phototransistor, einen Photowiderstands (LDR, light dependent resistor), einen Infrarotempfänger oder einen Piezosensor handeln.

Der Detektor kann das von der Quelle ausgesendete und vom Schaum im Prozessgefäss durch Reflexion und/oder Transmission veränderte Signal detektieren. Auf diese Weise können Signale mittels des Detektors nach Reflexion an einem Medium, insbesondere an aufkommendem Schaum, detektiert werden.

Das Verfahren kann weitere Schritte zur Reduktion eines Schaums bei Anwesenheit von Schaum umfassen, wie beispielsweise eine Druckerhöhung im Prozessgefäss und/oder eine Reduktion einer Prozesstemperatur, insbesondere der Temperatur im Prozessgefäss und/oder der Temperatur in einer Kühlvorrichtung, und/oder eine Veränderung der Bewegungsparameter des Prozessgefässes und/oder ein Einleiten eines Antischaummittels.

Ein beispielhaftes Messprinzip der Schaumerkennung ist nachfolgend ausgeführt. Ein Impulstimer schaltet eine IR-LED ein. Nach einer gewissen Einschwingzeit wird ein AD-Wandler getriggert und misst so das Signal vom Empfänger. Danach wird der Sender abgeschaltet. Je nach Ausführung sind ein Verstärker für den Sender und/oder ein Verstärker für den Empfänger notwendig. Das nach dem AD-Wandler zeitdiskrete Signal wird weiterverarbeitet. Mit Tiefpass TP1 wird der "DC Wert" eines ersten Messsignals ermittelt. Für ein zweites gefiltertes, effektives Messsignal wird der "DC Wert" aus dem ersten Messsignal abgezogen, quadriert und an einen zweiten Tiefpassfilter TP2 weitergeleitet. Die Quadrierung erzeugt ausschliesslich positive Werte und gewichtet grössere Signale stärker, wie beispielsweise ein RMS-Detektor. Der Tiefpass TP2 filtert hochfrequente Störanteile heraus und weist eine höhere Grenzfrequenz als der Tiefpass TP1 auf. Der Tiefpass TP1 filtert die sehr niederen Frequenzanteile heraus. Gleichzeitig wird ein dynamischer Schwellenwert ermittelt, der die Alterung des Sensors sowie Verunreinigungen und dergleichen kompensieren soll. Dazu wird das Messsignal mit einem weiteren Tiefpassfilter TP3 gefiltert und mit einer nichtlinearen Kennlinie bewertet. Der Tiefpassfilter TP3 ist wesentlich langsamer eingestellt als die Filter TP1 und TP2 und wird somit durch das gelegentliche Vorhandensein von Schaum nicht beeinflusst. Alternativ kann bei sehr dichtem Schaum zu einem Zeitpunkt, in welchem kein Schaum existiert (z.B. vor dem Destillationsstart), ein fixer Schwellenwert ermittelt werden, der dann für den gesamten Destillationsvorgang konstant bleibt. Es können auch beide Methoden kombiniert werden. Vom gefilterten, effektiven Messsignal wird der Schwellenwert abgezogen. Ist das Ergebnis der Subtraktion >0, ist Schaum vorhanden.

Ein beispielhaftes Messprinzip der Schaumerkennung mit Korrelator ist nachfolgend ausgeführt. Mit einem Korrelator kann die Empfindlichkeit gegenüber Störlicht bzw. Streulicht nochmals reduziert werden. Dazu wird ein Sendesignal während dem eingeschalteten Impuls mit einem Modulator in Form eines Modulationssignals moduliert. Dies kann ein einfacher Multiplikator sein. Das Modulationssignal sollte dabei in einem Frequenzbereich liegen, welche das Störlicht bzw. Streulicht möglichst nicht aufweist. Alternativ kann ein Modulationssignal mit breitem Frequenzanteil verwendet werden wie z.B. ein quasizufälliges Signal. Das modulierte Signal wird ausgesendet, wieder empfangen und mit einem AD-Wandler abgetastet. Die Samplefrequenz des AD-Wandlers sollte dabei mindestens doppelt so hoch sein wie der maximale Frequenzanteil im Modulationssignal. Das derart abgetastete Signal wird einem Kreuzkorrelator zugeführt, der es mit einer Kopie des Modulationssignals vergleicht. Je nach Impulslänge liefert der Kreuzkorrelator einen bis mehrere Peaks, die einem Maximalwertdetektor oder einem intelligenten Detektor zugeführt wird. Dabei sollte die Impulslänge mindestens zweimal so lang sein wie die Periodendauer des tiefsten Frequenzanteils im Modulationssignal. Das Ergebnis dieses Detektors entspricht proportional dem reflektierten Lichtsignal, wobei der Einfluss von Störlicht minimiert ist. Kreuzkorrelator und Detektor werden nach jedem Impuls wieder initialisiert.

Die Erfindung wird im Folgenden anhand von Abbildungen exemplarischer Ausführungsbeispiele näher erläutert. Es zeigen:
- Figur 1:: Eine schematische Darstellung einer Vorrichtung,
- Figur 2:: Eine Schaumerkennung mittels der Vorrichtung aus Figur 1,
- Figur 3a:: Perspektivische Darstellung eines Rotationsverdampfers mit der Vorrichtung im Schnitt entlang einer Achse
- Figur 3b:: Vergrösserte Darstellung der Anbringung der Vorrichtung.

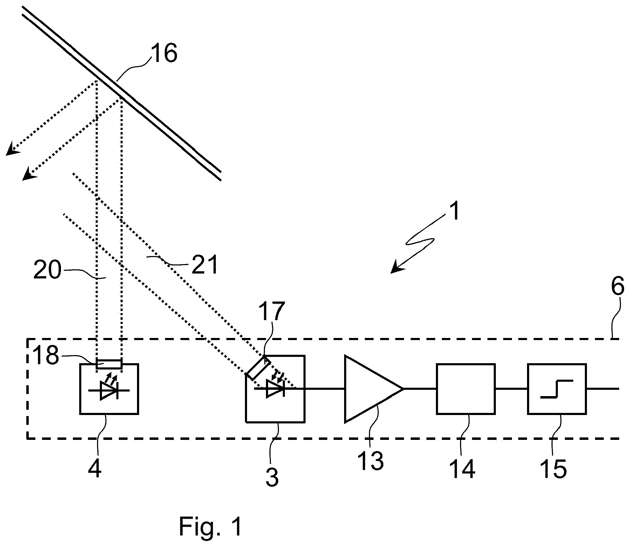

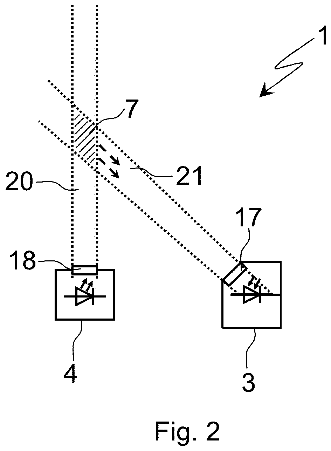

Figur 1 zeigt schematisch eine Vorrichtung des erfindungsgemässen Verdampfungssystems umfassend eine Quelle 4, in der Form einer LED, einen Detektor 3 in der Form einer Photodiode, einen Verstärker 13, einen Korrelator 14, und einen Komparator 15. Alternativ können die Elemente Verstärker 13, Korrelator 14 und Komparator 15 in einem internen Schaltkreis integriert und/oder deren Funktion mittels einer Software erfüllt werden. Die Vorrichtung 1 ist gekapselt in einem Glasstab 6 angeordnet und in einem Prozessgefäss anordenbar oder angeordnet. Der Quelle 4 ist eine Blende 18 vorgelagert, welche ein Aussenden eines definiert konzentrierten Signals 20, insbesondere eines Lichtstrahls, ermöglicht. Alternativ kann der Quelle 4 zusätzlich ein Filter und/oder eine Linsenoptik und/oder eine Spiegeloptik vorgelagert sein. Dem Detektor 3 ist eine Empfangsbegrenzung 17 vorgelagert. Alternativ kann dem Detektor 3 zusätzlich einer Blende und/oder einem Filter und/oder einer Linsenoptik und/oder einer Spiegeloptik vorgelagert sein. Die Empfangsbegrenzung 17 grenzt ein einfallendes Signal derart ein, dass die Detektion von Streustrahlung verringert wird, und fokussiert gegebenenfalls ein ankommendes Signal. Das von der Quelle 4 ausgesendete Signal 20, welches an einer Wand 16 des Prozessgefässes reflektiert wird, gelangt aufgrund gewählter geometrischer Einschränkungen nicht in den Detektor 3. Vielmehr wird das Signal in einen dem Detektor 3 abgewandten und entfernten Bereich geleitet. Trifft das von der Quelle 4 ausgesendete Signal 20 in einem Überschneidungsbereich mit einem in den Detektor einfallenden Signalweg 21 auf ein Medium 7 (vgl. Fig. 2), insbesondere auf einen Schaum, so wird das Signal an diesem Medium 7 reflektiert und kann vom Detektor 3 erfasst werden. Vom Detektor 3 erfasste Signale werden in den Verstärker 13 geleitet und verstärkt. Das verstärkte Signal kann direkt einem Komparator zugeführt werden, oder wird wie dargestellt vorgängig mittels eines Korrelators 14 oder eines Hochpasses gefiltert. Der Korrelator 14 und/oder der Komparator 15 können eine dynamische Schwelle aufweisen, welche beispielsweise eine Verschmutzung des Glasstabes kompensiert. Eine weitere Verarbeitung des Signals kann mittels einer Elektronik, welche ebenfalls im Glasstab integriert oder extern angeordnet sein kann, erfolgen. Anhand einer ermittelten Schaumerkennung können Schritte automatisiert und/oder manuell eingeleitet werden, die einer Schaumentwicklung entgegenwirken. Diese Schritte umfassen beispielsweise eine Druckerhöhung im Prozessgefäss, eine Reduktion einer Prozesstemperatur, insbesondere der Temperatur einer Heizvorrichtung, eine Veränderung der Rotationsgeschwindigkeit des Prozessgefässes und/oder ein Einleiten eines Antischaummittels

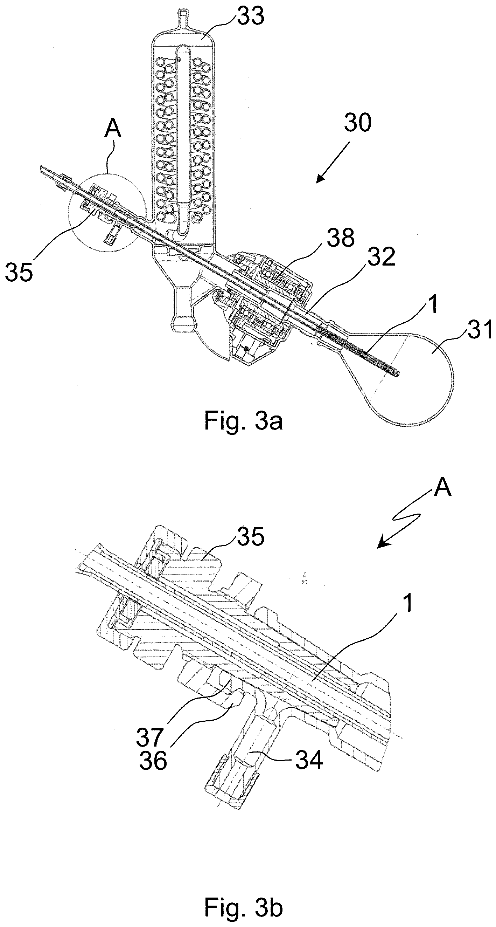

Figur 2 zeigt eine Schaumerkennung mittels der Vorrichtung 1 gemäss Figur 1, wobei lediglich die Quelle 4 und der Detektor 3 dargestellt sind. Die Quelle 4 sendet Signal 20 aus, welches durch die Blende 18 zu einem gerichtetem Strahl geformt wird. Dieses kann insbesondere in Form von gepulstem Infrarotlicht, vorzugsweise mit einer Wellenlänge von 850 nm, ausgesendet werden. Das ausgesendete Signal 20 trifft in einem Überschneidungsbereich mit einem in den Detektor einfallenden Signalweg 21 auf ein Medium 7, insbesondere auf einen Schaum. Das Signal 20 wird am Medium 7, insbesondere am Schaum, reflektiert und derart abgelenkt, dass es in den Detektor 3 gelangt. Dabei wird das in den Detektor 3 einfallende Signal von der vorgelagerten Empfangsbegrenzung 17 eingegrenzt. Der Detektor 3 weist einen Empfangsbereich von 730 bis 1010 nm auf. Anhand des erfassten Signals lässt sich eine Schaumentwicklung erkennen. Zudem lassen sich eine oder mehrere Differenzmessungen durchführen, um das Umgebungslicht und andere Störeinflüsse zu subtrahieren. Diese Referenzwerte können berücksichtigt werden und für die Auswertung der sich mit der Zeit verändernden Signaleigenschaften bei Schaumentwicklung herangezogen werden. Die Überwachung der Schaumentwicklung kann an einem oder mehreren Zeitpunkten oder kontinuierlich während eines Verdampfungsprozesses erfolgen. Figur 3a zeigt einen Rotationsverdampfer 30 umfassend ein Verdampfungsgefäss 31, eine Dampfdurchführung 32 und einen Flüssigkeitskühler 33, einen Einsatz 35 und eine Vorrichtung 1. Die Vorrichtung ist gekapselt in einem Stab, insbesondere einem durchsichtigem Stab, ausgestaltet. Die Vorrichtung 1 ist an einer Seite des Flüssigkeitskühlers 33 mittels des Einsatzes 35 (vgl. Figur 3b) am Rotationsverdampfer 30 statisch angebracht. Die Vorrichtung 1 reicht durch den Flüssigkeitskühler 33 und die Dampfdurchführung 32 bis in das Verdampfungsgefäss 31 hinein. Der Ausschnitt A zeigt die Elemente des Einsatzes 35 (vgl. Figur 3b).

Figur 3b zeigt die Anbringung der Vorrichtung 1 am Rotationsverdampfer 30 als vergrösserte Darstellung des Ausschnittes A (vgl. Figur 3a). Der Einsatz 35 ist an dem Rotationsverdampfer 30 angebracht und weist eine Aussparung, in welche die gekapselte Vorrichtung 1 eingeführt ist, auf. Der Einsatz 35 ist mittels eines Haltelements 36, welches um ein Haltemittel 37 des Rotationsverdampfers 30 (vgl. Figur 3a) greift, am Rotationsverdampfer fixiert. Die gekapselte Vorrichtung 1 kann innerhalb des Einsatzes 35 ausgerichtet und mittels eines Klemmelements 34, welches den Einsatz 35 einklemmt, innerhalb des Einsatzes fixiert werden.

## Patentansprüche

1. Verdampfungssystem (10) umfassend ein Prozessgefäss und eine Vorrichtung (1) zur Erkennung einer Schaumentwicklung mit einem Detektor (3) zum Erfassen eines elektromagnetischen und/oder akustischen Signals und einer Auswerteinheit zum Auswerten des vom Detektor (3) erfassten Signals, **dadurch gekennzeichnet, dass** die Vorrichtung (1) im Prozessgefäss anordenbar oder angeordnet ist.

2. Verdampfungssystem (10) nach Anspruch 1, wobei das Verdampfungssystem (10) eine Rotationsverdampfer ist.

3. Verdampfungssystem (10) nach Anspruch 1 oder 2, wobei die Vorrichtung (1) zusätzlich eine Quelle (4) zum Erzeugen des elektromagnetischen und/oder des akustischen Signals aufweist.

4. Verdampfungssystem (10) nach einem der Ansprüche 1 bis 3, wobei das Signal für das menschliche Auge und/oder das menschliche Gehör nicht wahrnehmbar ist.

5. Verdampfungssystem (10) nach einem der Ansprüche 1 bis 4, **dadurch gekennzeichnet, dass** die Quelle (4), der Detektor (3) und die Auswerteinheit in einer Einheit angeordnet sind.

6. Verdampfungssystem (10) nach Anspruch 5 **dadurch gekennzeichnet, dass** die Einheit stabförmig ausgestaltet ist.

7. Verdampfungssystem (10) nach einem der Ansprüche 3 bis 6, **dadurch gekennzeichnet, dass** die Quelle (4) und der Detektor (3) derart relativ zueinander angeordnet sind, dass der Detektor (3) ein von einem Medium (7) reflektiertes Signal und/oder ein durch das Medium (7) transmittiertes Signal detektiert.

8. Verdampfungssystem (10) nach einem der Ansprüche 1 bis 7, **dadurch gekennzeichnet, dass** eine Empfangsbegrenzung (17) zum Abschirmen eines unerwünschten Streusignals vor dem Detektor (3) angeordnet ist.

9. Verdampfungssystem (10) nach einem der Ansprüche 1 bis 8, **dadurch gekennzeichnet, dass** das Verdampfungssystem (10) Mittel aufweist, um Schaum im Prozessgefäss zu reduzieren.

10. Verfahren zum Erkennen einer Schaumentwicklung in einem Prozessgefäss eines Verdampfungssystems (10) gemäss einem der Ansprüche 1 bis 9, umfassend die Schritte
- Erfassen eines elektromagnetischen und/oder akustischen Signals mittels des im Prozessgefäss angeordneten Detektors (3) der Vorrichtung (1), und
- Auswerten des erfassten Signals mit der Auswerteinheit der Vorrichtung nach vorgegebenen Kriterien zur Erkennung einer Schaumentwicklung.

11. Verfahren nach Anspruch 10 zusätzlich umfassend den Schritt Aussenden eines elektromagnetischen und/oder akustischen Signals von einer Quelle (4), die im Prozessgefäss angeordnet ist.

12. Verfahren nach Anspruch 11, **dadurch gekennzeichnet, dass** die Quelle (4) ein Signal im Infrarotbereich, insbesondere in einem Wellenlängebereich von 700 bis 1400 nm, und/oder Schallwellen, insbesondere im Frequenzbereich von 20 KHz bis 1 GHz ausstrahlt.

13. Verfahren nach Anspruch 11 oder 12, **dadurch gekennzeichnet, dass** der Detektor (3) das von der Quelle (4) ausgesendete und von Schaum im Prozessgefäss durch Reflexion und/oder Transmission veränderte Signal detektiert.

14. Verfahren nach einem der Ansprüche 10 bis 13 zusätzlich umfassend den Schritt Reduktion eines Schaums bei Anwesenheit von Schaum.

15. Verfahren nach Anspruch 14, wobei die Reduktion eines Schaumes bei Anwesenheit von Schaum durch:
- Druckerhöhung im Prozessgefäss und/oder
- Reduktion einer Prozesstemperatur und/oder
- Veränderung der Bewegungsparameter des Prozessgefässes erfolgt.

## Claims

1. Evaporation system (10) comprising a process vessel and a device (1) for detecting foam development with a detector (3) for detecting an electromagnetic and/or acoustic signal and an evaluation unit for evaluating the signal detected by the detector (3), **characterised in that** the device (1) can be arranged or is arranged in the process vessel.

2. Evaporation system (10) according to claim 1, wherein the evaporation system (10) is a rotary evaporator.

3. An evaporation system (10) according to claim 1 or 2, wherein the device (1) additionally comprises a source (4) for generating the electromagnetic and/or the acoustic signal.

4. Vaporisation system (10) according to any one of claims 1 to 3, wherein the signal is not perceptible to the human eye and/or human hearing.

5. Evaporation system (10) according to one of claims 1 to 4, **characterised in that** the source (4), the detector (3) and the evaluation unit are arranged in one unit.

6. Evaporation system (10) according to claim 5, **characterised in that** the unit is rod-shaped.

7. An evaporation system (10) according to any one of claims 3 to 6, **characterized in that** the source (4) and the detector (3) are arranged relative to each other such that the detector (3) detects a signal reflected from a medium (7) and/or a signal transmitted through the medium (7).

8. An evaporation system (10) according to any one of claims 1 to 7, **characterized in that** a reception limiter (17) for shielding an unwanted stray signal is arranged in front of the detector (3).

9. An evaporation system (10) according to any one of claims 1 to 8, **characterised in that** the evaporation system (10) comprises means for reducing foam in the process vessel.

10. Method for detecting foam development in a process vessel of an evaporation system (10) according to one of claims 1 to 9, comprising the steps of
- detecting an electromagnetic and/or acoustic signal by means of the detector (3) of the device (1) arranged in the process vessel, and
- evaluating the detected signal with the evaluation unit of the device according to predetermined criteria for detecting foam development.

11. The method of claim 10 additionally comprising the step of emitting an electromagnetic and/or acoustic signal from a source (4) located in the process vessel.

12. Method according to claim 11, **characterised in that** the source (4) emits a signal in the infrared range, in particular in a wavelength range from 700 to 1400 nm, and/or sound waves, in particular in the frequency range from 20 KHz to 1 GHz.

13. Method according to claim 11 or 12, **characterized in that** the detector (3) detects the signal emitted by the source (4) and modified by foam in the process vessel by reflection and/or transmission.

14. The method of any one of claims 10 to 13 additionally comprising the step of reducing a foam in the presence of foam.

15. The process of claim 14, wherein the reduction of a foam in the presence of foam is achieved by:
- increasing the pressure in the process vessel and/or
- reduction of a process temperature and/or
- changing the movement parameters of the process vessel taking place.

## Revendications

1. Système d'évaporation (10) comprenant un récipient de traitement et un dispositif (1) pour détecter le développement de mousse avec un détecteur (3) pour détecter un signal électromagnétique et/ou acoustique et une unité d'évaluation pour évaluer le signal détecté par le détecteur (3), **caractérisé en ce que** le dispositif (1) peut être ou est disposé dans le récipient de traitement.

2. Système d'évaporation (10) selon la revendication 1, dans lequel le système d'évaporation (10) est un évaporateur rotatif.

3. Système d'évaporation (10) selon la revendication 1 ou 2, dans lequel ledit dispositif (1) comprend en outre une source (4) pour générer ledit signal électromagnétique et/ou ledit signal acoustique.

4. Système d'évaporation (10) selon l'une quelconque des revendications 1 à 3, dans lequel le signal est imperceptible à l'oeil humain et/ou à l'audition humaine.

5. Système d'évaporation (10) selon l'une quelconque des revendications 1 à 4, **caractérisé en ce que** la source (4), le détecteur (3) et l'unité d'évaluation sont disposés en une seule unité.

6. Système d'évaporation (10) selon la revendication 5, **caractérisé en ce que** l'unité est en forme de tige.

7. Système d'évaporation (10) selon l'une quelconque des revendications 3 à 6, **caractérisé en ce que** la source (4) et le détecteur (3) sont disposés l'un par rapport à l'autre de telle sorte que le détecteur (3) détecte un signal réfléchi par un milieu (7) et/ou un signal transmis par le milieu (7) .

8. Système d'évaporation (10) selon l'une quelconque des revendications 1 à 7, **caractérisé en ce qu'**un limiteur de réception (17) destiné à faire écran à un signal parasite indésirable est disposé devant le détecteur (3).

9. Système d'évaporation (10) selon l'une quelconque des revendications 1 à 8, **caractérisé en ce que** le système d'évaporation (10) comprend des moyens pour réduire la mousse dans le récipient de traitement.

10. Procédé pour détecter le développement de mousse dans un récipient de traitement d'un système d'évaporation (10) selon l'une des revendications 1 à 9, comprenant les étapes suivantes
- détecter un signal électromagnétique et/ou acoustique au moyen du détecteur (3) du dispositif (1) disposé dans le récipient de traitement, et
- évaluer le signal détecté au moyen de l'unité d'évaluation de l'appareil selon des critères prédéterminés afin de détecter un développement de mousse.

11. Procédé selon la revendication 10, comprenant en outre l'étape consistant à émettre un signal électromagnétique et/ou acoustique à partir d'une source (4) disposée dans le récipient de traitement.

12. Procédé selon la revendication 11, **caractérisé en ce que** la source (4) émet un signal dans le domaine infrarouge, notamment dans une plage de longueur d'onde de 700 à 1400 nm, et/ou des ondes sonores, notamment dans la plage de fréquence de 20 KHz à 1 GHz.

13. Procédé selon la revendication 11 ou 12, **caractérisé en ce que** le détecteur (3) détecte le signal émis par la source (4) et modifié par la mousse dans le récipient de traitement par réflexion et/ou transmission.

14. Procédé de l'une quelconque des revendications 10 à 13 comprenant en outre l'étape consistant à réduire une mousse en présence de mousse.

15. Procédé de la revendication 14, dans lequel la réduction d'une mousse en présence d'une mousse est obtenue par:
- l'augmentation de la pression dans le récipient de traitement et/ou
- réduction la température du processus et/ou
- modification des paramètres d'agitation du récipient de traitement.
